# EUROPÄISCHE PATENTSCHRIFT

(11) **EP 0 845 010 B1**
(45) Veröffentlichungstag und Bekanntmachung des Hinweises auf die Patenterteilung: **15.12.1999**
(21) Anmeldenummer: 96927061.0
(22) Anmeldetag: 30.07.1996
(51) Int. Cl.: C08G 14/10

(54) **MODIFIZIERTE MELAMIN-FORMALDEHYD-HARZE**
MODIFIED MELAMINE FORMALDEHYDE RESINS
RESINES MODIFIEES MELAMINE-FORMALDEHYDE

(30) Priorität: 17.08.1995 DE 19530178
(43) Veröffentlichungstag der Anmeldung: 03.06.1998
(73) Patentinhaber: BASF AKTIENGESELLSCHAFT, 67056 Ludwigshafen (DE)
(72) Erfinder: GUENTHER, Erhard, D-67454 Ha loch (DE); REUTHER, Wolfgang, D-69118 Heidelberg (DE)
(86) Internationale Anmeldenummer: EP9603352
(87) Internationale Veröffentlichungsnummer: WO9707149

(56) Entgegenhaltungen:
- EP-A- 0 523 485
- DE-A- 2 915 457
- JP-A- 61 236 834

## Beschreibung

Die Erfindung betrifft die Verwendung von Kondensationsprodukten, erhältlich durch Kondensation eines Gemisches, enthaltend als wesentliche Komponenten
(A) 90 bis 99,9 Mol-% eines Gemisches bestehend im wesentlichen aus
   (a) 30 bis 100 Mol-% Melamin und
   (b) 0 bis 70 Mol-% eines substituierten Melamins der allgemeinen Formel I 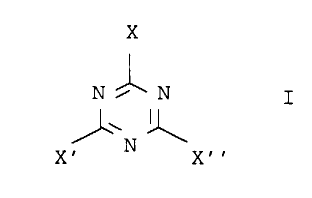 in der X, X' und X" ausgewählt sind aus der Gruppe bestehend aus -NH₂, -NHR und -NRR', und X, X' und X" nicht gleichzeitig -NH2 sind, und R und R' ausgewählt sind aus der Gruppe bestehend aus Hydroxy-C₂-C₁₀-alkyl, Hydroxy-C₂-C₄-alkyl-(oxa-C₂-C₄-alkyl)ₙ, mit n = 1 bis 5, und Amino-C₂-C₁₂-alkyl, oder Mischungen von Melaminen I, und
   c) 1 bis 70 Mol-%, bezogen auf (a) + (b), eines substituierten Triazins der allgemeinen Formel II 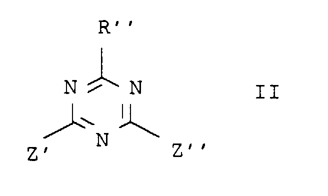 in der R" für Methyl oder Phenyl steht, und Z' und Z" ausgewählt sind aus der Gruppe bestehend aus -NH₂, -NHR^{III} und NR^{III}R^{IV}, und R^{III} und R^{IV} ausgewählt sind aus der Gruppe bestehend aus Hydroxy-C₂-C₁₀-alkyl, Hydroxy-C₂-C₄-alkyl-(oxa-C₂-C₄-alkyl)ₙ, mit n = 1 bis 5, -CH₂CH₂-S-CH₂CH₂OH, und Amino-C₂-C₁₂-alkyl, oder Mischungen der Triazine II, und
B) 0,1 bis 10 Mol-%, bezogen auf (A) (a), (A) (b) und (B), unsubstituierten oder mit Resten, ausgewählt aus der Gruppe aus C₁-C₉-Alkyl und Hydroxy, substituierten Phenolen, mit zwei oder drei Phenolgruppen substituierten C₁-C₄-Alkanen, Di(hydroxyphenyl)sulfone oder Mischungen dieser Phenole,
mit
Formaldehyd oder Formaldehyd-lieferenden Verbindungen, wobei das Molverhältnis von Melamin, substituierten Melamin I und Triazin II zu Formaldehyd im Bereich von 1:1,15 bis 1:4,5 liegt, zur Herstellung von Fasern und Schaumstoffen.

Ferner betrifft die Erfindung Fasern und Schaumstoffe, erhältlich aus dieser Verwendung.

Die EP-A-523 485 beschreibt Kondensationsprodukte, erhältlich durch Kondensation eines Gemisches, enthaltend als wesentliche Komponenten
(A) 90 bis 99,9 Mol-% eines Gemisches bestehend im wesentlichen aus
   (a) 30 bis 99 Mol-% Melamin und
   (b) 1 bis 70 Mol-% eines substituierten Melamins der allgemeinen Formel I 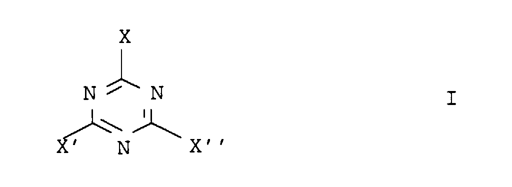 in der X, X' und X" ausgewählt sind aus der Gruppe bestehend aus -NH₂, -NHR und -NRR', und X, X' und X" nicht gleichzeitig -NH₂ sind, und R und R' ausgewählt sind aus der Gruppe bestehend aus Hydroxy-C₂-C₄-alkyl-, Hydroxy-C₂-C₄-alkyl-(oxa-C₂-C₄-alkyl)ₙ, mit n = 1 bis 5, und Amino-C₂-C₁₂-alkyl, oder Mischungen von Melaminen I, und
(B) 0,1 bis 10 Mol-%, bezogen auf (A) und (B), unsubstituierten oder mit Resten, ausgewählt aus der Gruppe aus C₁-C₉-Alkyl und Hydroxy, substituierten Phenolen, mit zwei oder drei Phenolgruppen substituierten C₁-C₄-Alkanen, Di(hydroxyphenyl)sulfone oder Mischungen dieser Phenole,
mit
Formaldehyd oder Formaldehyd-liefernden Verbindungen, wobei das Molverhältnis von Melaminen zu Formaldehyd im Bereich von 1:1,15 bis 1:4,5 liegt, sowie deren Verwendung zur Herstellung von Fasern und Schaumstoffen und Formkörper, erhältlich aus diesen Produkten. Nachteilig an den Fasern der EP-A 523 485 ist, daß sie eine zu geringe Dehnung aufweisen.

JP-A-61 236 834 beschreibt Laminate, wie sie als Trägermaterial für elektrische Schaltkreise verwendet werden können, aus (A) einem Gemisch aus (a) Melamin und (c) substituierten Triazinen der allgemeinen Formel II und (B) Phenolen mit Formaldehyd.

Der Erfindung lag daher die Aufgabe zugrunde, Fasern und Schaumstoffe aus Melamin-Formaldehyd-Kondensationsprodukten bereitzustellen, die in ausgehärteter Form eine verbesserte Faserdehnung aufweisen.

Demgemäß wurde die eingangs definierte Verwendung gefunden.

Außerdem wurden Fasern und Schaumstoffe, erhältlich aus dieser Verwendung, gefunden.

Die erfindungsgemäß verwendeten Melamin-Harze enthalten als Monomerbaustein (A) 90 bis 99,9 Mol-% eines Gemisches bestehend im wesentlichen aus 30 bis 100, bevorzugt 50 bis 99, besonders bevorzugt 85 bis 95 Mol-% Melamin und 0 bis 70, bevorzugt 1 bis 50, besonders bevorzugt 5 bis 15 Mol-% eines substituierten Melamins I oder Mischungen substituierter Melamine I, sowie 1 bis 70, bevorzugt von 1 bis 40, besonders bevorzugt von 1 bis 25 Mol-%, bezogen auf (a) + (b), eines substituierten Triazins II.

Als weiteren Monomerbaustein (B) enthalten die Melamin-Harze 0,1 bis 10 Mol-%, bezogen auf die Gesamtmolzahl an Monomerbausteinen (A) (a), (A) (b) und (B), eines Phenols oder eines Gemisches von Phenolen.

Die erfindungsgemäß verwendeten Kondensationsprodukte sind erhältlich durch Umsetzung der Komponenten (A) und (B) mit Formaldehyd oder Formaldehyd-liefernden Verbindungen, wobei das Molverhältnis von Melamin, substituierten Melamin I und Triazin II zu Formaldehyd im Bereich von 1:1,15 bis 1:4,5, bevorzugt von 1:1,8 bis 1:3,0 liegt.

Als substituierte Melamine der allgemeinen Formel I 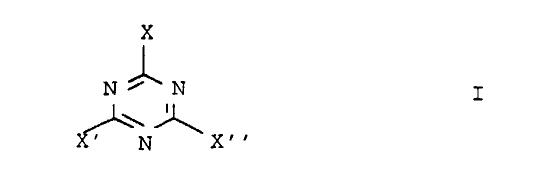 kommen solche in Betracht, in denen X, X' und X" ausgewählt sind aus der Gruppe bestehend aus -NH₂, -NHR und -NRR', wobei X, X' und X" nicht gleichzeitig -NH₂ sind, und R und R' ausgewählt sind aus der Gruppe bestehend aus Hydroxy-C₂-C₁₀-alkyl, Hydroxy-C₂-C₄-alkyl-(oxa-C₂-C₄-alkyl)ₙ, mit n = 1 bis 5, und Amino-C₂-C₁₂-alkyl.

Als Hydroxy-C₂-C₁₀-alkyl-Gruppen wählt man bevorzugt Hydroxy-C₂-C₆-alkyl wie 2-Hydroxyethyl, 3-Hydroxy-n-propyl, 2-Hydroxyisopropyl, 4-Hydroxy-n-butyl, 5-Hydroxy-n-pentyl, 6-Hydroxy-n-hexyl, 3-Hydroxy-2,2-dimethylpropyl, bevorzugt Hydroxy-C₂-C₄-alkyl wie 2-Hydroxyethyl, 3-Hydroxy-n-propyl, 2-Hydroxyisopropyl und 4-Hydroxy-n-butyl, besonders bevorzugt 2-Hydroxyethyl und 2-Hydroxyisopropyl.

Als Hydroxy-C₂-C₄-alkyl-(oxa-C₂-C₄-alkyl)ₙ-Gruppen wählt man bevorzugt solche mit n = 1 bis 4, besonders bevorzugt solche mit n = 1 oder 2 wie 5-Hydroxy-3-oxa-pentyl, 5-Hydroxy-3-oxa-2,5-dimethyl-pentyl, 5-Hydroxy-3-oxa-1,4-dimethyl-pentyl, 5-Hydroxy-3-oxa-1,2,4,5-tetramethyl-pentyl, 8-Hydroxy-3,6-dioxa-octyl.

Als Amino-C₂-C₁₂-alkyl-Gruppen kommen bevorzugt Amino-C₂-C₈-alkyl-Gruppen wie 2-Aminoethyl, 3-Aminopropyl, 4-Aminobutyl, 5-Aminopentyl, 6-Aminohexyl, 7-Aminoheptyl sowie 8-Aminooctyl, besonders bevorzugt 2-Aminoethyl und 6-Aminohexyl, ganz besonders bevorzugt 6-Aminohexyl, in Betracht.

Für die Erfindung besonders geeignete substituierte Melamine I sind folgende Verbindungen:
mit der 2-Hydroxyethylamino-Gruppe substituierte Melamine wie 2-(2-Hydroxyethylamino)-4,6-diamino-1,3,5-triazin, 2,4-Di-(2-hydroxyethylamino)-6-amino-1,3,5-triazin, 2,4,6-Tris-(2-hydroxyethylamino)-1,3,5-triazin, mit der 2-Hydroxyisopropylamino-Gruppe substituierte Melamine wie 2-(2-Hydroxyisopropylamino)-4,6-diamino-1,3,5- triazin, 2,4-Di-(2-hydroxyisopropylamino)-6-amino-1,3,5-triazin, 2,4,6-Tris-(2-hydroxyisopropylamino)-1,3,5-triazin, mit der 5-Hydroxy-3-oxa-pentylamino-Gruppe substituierte Melamine wie 2-(5-Hydroxy-3-oxa-pentylamino)-4,6-diamino-1,3,5-triazin, 2,4-Di-(5-hydroxy-3-oxa-pentylamino)-6-amino-1,3,5-triazin, 2,4,6-Tris-(5-hydroxy-3-oxa-pentylamino)-1,3,5-triazin, mit der 6-Aminohexylamino-Gruppe substituierte Melamine wie 2-(6-Aminohexylamino)-4,6-diamino-1,3,5-triazin, 2,4-Di-(6-aminohexylamino)-6-amino-1,3,5-triazin, 2,4,6-Tris-(6-aminohexylamino)-1,3,5-triazin oder Gemische dieser Verbindungen, beispielsweise ein Gemisch aus 10 Mol-% 2-(5-Hydroxy-3-oxapentylamino)-4,6-diamino-1,3,5-triazin, 50 Mol-% 2,4-Di-(5-hydroxy-3-oxa-pentylamino)-6-amino-1,3,5-triazin und 40 Mol-% 2,4,6-Tris-(5-hydroxy-3-oxa-pentylamino)-1,3,5-triazin.

Als substituierte Triazine der allgemeinen Formel II 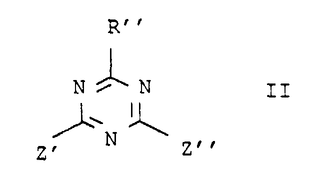 kommen solche in Betracht, in denen R" für Methyl oder Phenyl steht und Z' und Z" ausgewählt sind aus der Gruppe, bestehend aus - NH₂, -NHR^{III} und -NR^{III} R^{IV}, und R^{III} und R^{IV} ausgewählt sind aus der Gruppe, bestehend aus Hydroxy-C₂-C₁₀-alkyl, Hydroxy-C₂-C₄-alkyl-(oxa-C₂-C₄-alkyl)ₙ, mit n = 1 bis 5, -CH₂CH₂-S-CH₂CH₂OH, und Amino-C₂-C₁₂-alkyl.

Als Hydroxy-C₂-C₁₀-alkyl-Gruppen wählt man bevorzugt Hydroxy-C₂-C₆-alkyl wie 2-Hydroxyethyl, 3-Hydroxy-n-propyl, 2-Hydroxyisopropyl, 4-Hydroxy-n-butyl, 5-Hydroxy-n-pentyl, 6-Hydroxy-n-hexyl, 3-Hydroxy-2, 2-dimethylpropyl, bevorzugt Hydroxy- C₂- C₄-alkyl wie 2-Hydroxyethyl, 3-Hydroxy-n-propyl, 2-Hydroxyisopropyl und 4-Hydroxy-n-butyl, besonders bevorzugt 2-Hydroxyethyl und 2-hydroxyisopropyl.

Als Hydroxy-C₂-C₄-alkyl-(oxa-C₂-C₄-alkyl)ₙ-Gruppen wählt man bevorzugt solche mit n = 1 bis 4, besonders bevorzugt solche mit n= 1 oder 2 wie 5-Hydroxy-3-oxa-pentyl, 5-Hydroxy-3-oxa-2,5-dimethyl-pentyl, 5-Hydroxy-3-oxa-1,4-dimethyl-pentyl, 5-Hydroxy-3-oxa-1,2,4,5-tetramethyl-pentyl, 8-Hydroxy-3,6-dioxaoctyl.

Als Amino-C₂-C₁₂-alkyl-Gruppen kommen bevorzugt Amino-C₂-C₈-alkyl-Gruppen wie 2-Aminoethyl, 3-Aminopropyl, 4-Aminobutyl, 5-Aminopentyl, 6-Aminohexyl, 7-Aminoheptyl sowie 8-Aminooctyl, besonders bevorzugt 2-Aminoethyl und 6-Aminohexyl, ganz besonders bevorzugt 6-Aminohexyl, in Betracht.

Für die Erfindung besonders geeignete substituierte Triazine II sind folgende Verbindungen:
2,4-(Di-5-hydroxy-3-oxapentylamin)-6-methyl-1,3,5-triazin,
2,4-(Di-5-hydroxy-3-thiopentylamin)-6-methyl-1,3,5-triazin,
2,4-(Di-5-hydroxy-3-oxapentylamin)-6-phenyl-1,3,5-triazin,
2,4-(Di-5-hydroxy-3-thiopentylamin)-6-phenyl-1,3,5-triazin,
2,4-(Di-5-hydroxy-3-oxaethylamin)-6-methyl-1,3,5-triazin,
2,4-(Di-5-hydroxy-3-thioethylamin)-6-methyl-1,3,5-triazin,
2,4-(Di-5-hydroxy-3-oxaethylamin)-6-phenyl-1,3,5-triazin,
2,4-(Di-5-hydroxy-3-thioethylamin)-6-phenyl-1,3,5-triazin.

Die substituierten Triazine II sind erhältlich durch Aminaustausch der entsprechenden in 6-Position substituierten 2,4-Diamino-1,3,5-triazine mit den entsprechenden primären Aminen R^{III}NH und R^{IV}NH₂. Üblicherweise führt man den Aminaustausch bei Temperaturen im Bereich von 100 bis 220, vorzugsweise von 120 bis 200°C durch, wobei der Druck zweckmäßig Atmosphärendruck ist.

Man kann die Reaktion in Gegenwart von Lösungsmitteln, bevorzugt Polyolen, wie Ethylenglykol, 1,2-Propylenglykol, Diethylenglykol, oder Triethylenglykol, durchführen.

Das Molverhältnis von Amin, R^{III}NH₂ bzw. R^{IV}NH₂, zu Triazin wählt man üblicherweise im Bereich von 3,0:1 bis 8,0:1, vorzugsweise von 4,0:1 bis 5,0:1. Besonders bevorzugt ist die Verfahrensweise, in der man das Amin im Überschuß einsetzt, so daß auf die weitere Zugabe eines Lösungsmittels verzichtet werden kann.

In einer bevorzugten Ausführungsform führt man den Aminoaustausch in Gegenwart von sauren Katalysatoren durch, insbesondere mit starken und mittelstarken Protonsäuren wie Flußsäure, Salzsäure, Bromwasserstoffsäure, Schwefelsäure, Salpetersäure, Phosphorsäure, Amidosulfonsäure, Thiocyansäure, p-Toluolsulfonsäure oder Methansulfonsäure sowie Lewis-Säuren wie Bortrifluorid, Aluminiumchlorid, Zinn(IV)chlorid, Antimon(V)fluorid oder Eisen(III)bromid.

Die Anwesenheit eines Katalysators ist bei Guanaminen nach bisherigen Beobachtungen jedoch nicht zwingend erforderlich.

Zweckmäßig verfolgt man den Reaktionsverlauf mit analytischen Methoden, wobei die HPLC bevorzugt eingesetzt werden kann.

Falls man den Aminoaustausch in Gegenwart eines der zuvorgenannten Katalysatoren durchführt, neutralisiert man in der Regel zur Isolierung der Triazine II mit einer üblichen Base wie ein Alkalimetallhydroxid, insbesondere Natriumhydroxid oder Kaliumhydroxid, und trennt dann die ausgefallenen Salze ab.

Überschüssiges Amin kann man bei vermindertem Druck (0,1 bis 100 mbar), (bevorzugt 10 bis 20 mbar) bei einer Temperatur im Bereich von 100 bis 300, vorzugsweise von 100 bis 200°C, je nach Siedepunkt des verwendeten Amins, abdestillieren.

Als Phenole (B) eignen sich ein oder zwei Hydroxygruppen enthaltende Phenole wie unsubstituierte oder mit Resten, ausgewählt aus der Gruppe aus C₁-C₉-Alkyl und Hydroxy, substituierte Phenole sowie mit zwei oder drei Phenolgruppen substituierte C₁-C₄-Alkane, Di(hydroxyphenyl)sulfone oder Mischungen dieser Phenole.

Als bevorzugte Phenole kommen in Betracht Phenol, 4-Methyl-phenol, 4-tert.-Butyl-phenol, 4-n-Octyl-phenol, 4-n-Nonyl-phenol, Brenzcatechin, Resorcin, Hydrochinon, 2,2-Bis(4-hydroxyphenyl)propan, 4,4'-Dihydroxydiphenylsulfon, besonders bevorzugt Phenol, Resorcin und 2,2-Bis(4-hydroxyphenyl)propan.

Formaldehyd setzt man in der Regel als wäßrige Lösung mit einer Konzentration von zum Beispiel 40 bis 50 Gew.-% oder in Form von bei der Umsetzung mit (A) und (B) Formaldehyd liefernden Verbindungen, beispielsweise als oligomeren oder polymeren Formaldehyd in fester Form wie Paraformaldehyd, 1,3,5-Trioxan oder 1,3,5,7-Tetroxocan ein.

Für die Herstellung von Fasern verwendet man zweckmäßig 1 bis 50, vorzugsweise 5 bis 15 und insbesondere 7 bis 12 Mol-% des substituierten Melamins I, 1 bis 25 mol-% substituiertes Triazin II, sowie 0,1 bis 9,5, bevorzugt 1 bis 5 Mol-% eines der oben angeführten Phenole oder Mischungen derselben.

Für die Herstellung von Schaumstoffen verwendet man zweckmäßig 0,5 bis 20, vorzugsweise 1 bis 10 und insbesondere 1,5 bis 5 Mol-% des substituierten Melamins I, 1 bis 25 mol-% substituiertes Triazin II oder Mischungen davon, sowie 0,1 bis 5, bevorzugt 1 bis 3 Mol-% eines der oben angeführten Phenole oder Mischungen davon.

Zur Herstellung der Harze polykondensiert man Melamin, substituiertes Melamin I, substituiertes Triazin II und Phenol zusammen mit Formaldehyd bzw. Formaldehyd-liefernden Verbindungen. Man kann dabei alle Komponenten gleich zu Beginn vorlegen oder man kann sie portionsweise und sukzessive zu Reaktion bringen und den dabei gebildeten Vorkondensaten nachträglich weiteres Melamin, substituiertes Melamin oder Phenol zufügen.

Die Polykondensation führt man in der Regel in an sich bekannter Weise durch (s. EP-A 355 760, Houben-Weyl, Bd.14/2, S.357 ff).

Die Reaktionstemperaturen wählt man dabei im allgemeinen in einem Bereich von 20 bis 150, bevorzugt von 40 bis 140°C.

Der Reaktionsdruck ist in der Regel unkritisch. Man arbeitet im allgemeinen in einem Bereich von 100 bis 500 kPa, bevorzugt 100 bis 300 kPa.

Man kann die Reaktion mit oder ohne Lösungsmittel durchführen. In der Regel setzt man beim Einsatz von wäßriger Formaldehydlösung kein Lösungsmittel zu. Bei Verwendung von Formaldehyd gebunden in fester Form wählt man als Lösungsmittel üblicherweise Wasser, wobei die verwendete Menge in der Regel im Bereich von 5 bis 40, bevorzugt von 15 bis 25 Gew.-%, bezogen auf die Gesamtmenge an eingesetzten Monomeren, liegt.

Ferner führt man die Polykondensation im allgemeinen in einem pH-Bereich oberhalb von 6 aus. Bevorzugt ist der pH-Bereich von 7,5 bis 10,0, besonders bevorzugt von 8 bis 10.

Des weiteren kann man dem Reaktionsgemisch geringe Mengen üblicher Zusätze wie Alkalimetallsulfiten, z.B. Natriumdisulfit und Natriumsulfit, Alkalimetallformiaten, z.B. Natriumformiat, Alkalimetallcitrate, z.B. Natriumcitrat, Phosphate, Polyphosphate, Harnstoff, Dicyandiamid oder cyanamid hinzufügen. Man kann sie als reine Einzelverbindungen oder als Mischungen untereinander jeweils in Substanz oder als wäßrige Lösungen vor, während oder nach der Kondensationsreaktion zusetzen.

Andere Modifizierungsmittel sind Amine sowie Aminoalkohole wie Diethylamin, Ethanolamin, Diethanolamin oder 2-Diethylaminoethanol.

Als weitere Zusatzstoffe kommen Füllstoffe, Emulgatoren oder Treibmittel in Betracht.

Als Füllstoffe kann man beispielsweise faser- oder pulverförmige anorganische Verstärkungsmittel oder Füllstoffe wie Glasfasern, Metallpulver, Metallsalze oder Silikate, z.B. Kaolin, Talkum, Schwerspat, Quarz oder Kreide, ferner Pigmente und Farbstoffe einsetzen. Als Emulgatoren verwendet man in der Regel die üblichen nichtionogenen, anionenaktiven oder kationaktiven organischen Verbindungen mit langkettigen Alkylresten. Bei der Verarbeitung der nicht ausgehärteten Harze zu Schäumen kann man als Treibmittel beispielsweise Pentan einsetzen.

Die Polykondensation kann man diskontinuierlich oder kontinuierlich, beispielsweise in einem Extruder (s. EP-A 355 760), nach an sich bekannten Methoden durchführen.

Die Herstellung von Formkörpern durch Härtung der erfindungsgemäßen Kondensationsprodukte erfolgt in üblicher Weise durch Zusatz von geringen Mengen an Säuren wie Ameisensäure, Schwefelsäure oder Ammoniumchlorid.

Schaumstoffe können hergestellt werden, in dem man eine wäßrige Lösung oder Dispersion, welche das noch nicht ausgehärtete Kondensat, einen Emulgator, ein Treibmittel und einen Härter, sowie gegebenenfalls übliche Zusatzstoffe, wie oben aufgeführt, enthält, verschäumt und anschließend den Schaum aushärtet. Ein solches Verfahren ist in der DE-A 29 15 457 eingehend beschrieben.

Zur Herstellung von Fasern verspinnt man in der Regel das erfindungsgemäß verwendete Melamin-Harz in an sich bekannter Weise beispielsweise nach Zusatz eines Härters bei Raumtemperatur in einer Rotationsspinnapparatur und härtet anschließend die Rohfasern in einer erhitzten Atmosphäre aus, oder man verspinnt in einer erhitzten Atmosphäre, verdampft dabei gleichzeitig das als Lösungsmittel dienende Wasser und härtet das Kondensat aus. Ein solches Verfahren ist in der DE-A 23 64 091 eingehend beschrieben.

Der Vorteil der erfindungsgemäßen Verwendung liegt in der Bereitstellung von Melamin-Formaldehyd-Fasern mit verbesserten Faserdehnungseigenschaften.

### Beispiele

Zur Bestimmung des Gewichtsverlustes durch Hydrolyse wurden die Harze 24 h siedendem Wasser (100°C) ausgesetzt. Dabei wurde vor und nach der Hydrolyse das Harz gewogen und aus der Differenz der Meßwerte und dem Anfangsgewicht der (relative) Gewichtsverlust errechnet.

Die Bruchfestigkeit (tenacity) und die Dehnung (elongation) wurden gemäß PM-T 4001-82 bestimmt.

### Beispiel 1 (Herstellung Triazin II)

500,56 g Acetoguanamin, 1682,4 g Aminoethoxyethanol und 106,8 g Ammoniumchlorid wurden 60 h bei 175°C gerührt. Der Fortgang der Reaktion wurde per HPLC (C18-Säule 5 µm, Laufmittel: 1 g KHPO₄/237 g MeOH/700 g H₂O) verfolgt. Anschließend wurde auf 80°C abgekühlt und mit Natronlauge (50%ig) das eingesetzte Ammoniumchlorid neutralisiert. Entstandenes Natriumchlorid wurde abgesaugt, der in der Lösung verbleibende Aminüberschuß abdestilliert (170°C/15 mbar). Es fielen 1170 g farbloses Harz (91,4 %), bestehend aus 2,4 Di-(5-hydroxy-3-oxapentylamin)-6-methyl-1,3,5-triazin an.

### Beispiel 2 (Harz mit Triazin I und Triazin II)

1663 g Melamin, 678,65 g einer 80%igen Mischung aus 10 Mol-% 2-(5-hydroxy-3-oxypentylamin)-4,6-diamino-1,3,5-triazin, 50 Mol-% 2,4-Di(5-hydroxy-3-oxapentylamine)-6-amino-1,3,5-triazin und 40 Mol-% 2,4,6-Tri(5-hydroxy-3-oxapentylamine-1,3,5-triazin in Wasser ("HOM"), 206,42 g Acetoguanamin, 1207 g Formaldehyd (40%ig), 507,2 g Paraformaldehyd, 37,62 g Bisphenol A und 8,25 g Diethylethanolamin wurden bei 98°C 140 min bis zu einer Viskosität von 470 Pas gerührt. Anschließend wurde durch Eiskühlung auf RT abgekühlt. Nach Zugabe von 1 %-iger Ameisensäure wurde das Harz in bekannter Weise (siehe EP-A 523 485) zu Fasern versponnen.
AATCC: 205 ppm (Formaldehyd-Gehalt)
Gewichtsverlust durch Hydrolyse (24 h bei 100°C): 2,8 %
Festigkeit: 388 N/mm²
Dehnung:32,7 %

### Beispiel 3 (Harz nur mit Triazin II)

1871,1 g Melamin, 496,7 g Triazin II aus Beispiel 1, 1414,7 g 40%ige wäßriges Formaldehyd, 424,1 g Paraformaldehyd, 37,6 g Bisphenol A und 8,25 ml Diethylethanolamin wurden 142 min bei 98°C bis zu einer Viskosität von 450 Pas gerührt. Anschließend wurde rasch auf RT abgekühlt. Nach Zugabe von 1 %-iger Ameisensäure wurde das Harz in bekannter Weise (siehe Bsp. 2) zu Fasern versponnen.
AATCC: 125 ppm
Gewichtsverlust durch Hydrolyse (24 h bei 100°C): 2,3 %
Festigkeit: 558 N/mm²
Dehnung: 30 %

### Vergleichsbeispiel (Harz ohne Triazin II)

Eine Mischung aus 1871 g Melamin, 620 g einer 80gew.-%igen HOM-Mischung (siehe Bsp. 2), 472,8 g Paraformaldehyd, 38,2 g Phenol und 15,4 ml Diethylethanolamin wurden innerhalb von 150 min bei 98°C bis zu einer Viskosität von 500 Pas kondensiert. Nach Zugabe von 1 %-iger Ameisensäure wurde das Harz in bekannter Weise (siehe Bsp. 2) zu Fasern versponnen.
AATCC: 253 ppm
Gewichtsverlust durch Hydrolyse: 2,5 %
Festigkeit: 427 N/mm²
Dehnung: 21 %

## Patentansprüche

1. Verwendung von Kondensationsprodukten, erhältlich durch Kondensation eines Gemisches, enthaltend als wesentliche Komponenten
(A) 90 bis 99,9 Mol-% eines Gemisches bestehend im wesentlichen aus
(a) 30 bis 100 Mol-% Melamin und
(b) 0 bis 70 Mol-% eines substituierten Melamins der allgemeinen Formel I 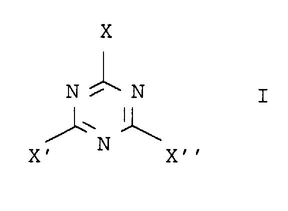 in der X, X' und X" ausgewählt sind aus der Gruppe bestehend aus -NH₂, -NHR und -NRR', und X, X' und X" nicht gleichzeitig -NH₂ sind, und R und R' ausgewählt sind aus der Gruppe bestehend aus Hydroxy-C₂-C₁₀-alkyl, Hydroxy-C₂-C₄-alkyl(oxa-C₂-C₄-alkyl)ₙ, mit n = 1 bis 5, und Amino-C₂-C₁₂-alkyl, oder Mischungen von Melaminen I, und
c) 1 bis 70 Mol-%, bezogen auf (a) + (b), eines substituierten Triazins der allgemeinen Formel II 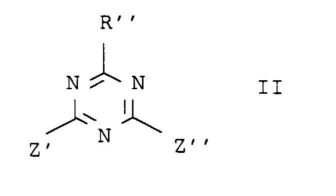 in der R" für Methyl oder Phenyl steht, und Z' und Z" ausgewählt sind aus der Gruppe bestehend aus -NH₂, -NHR^{III} und NR^{III}R^{IV}, und R^{III} und R^{IV} ausgewählt sind aus der Gruppe bestehend aus Hydroxy-C₂-C₁₀-alkyl, Hydroxy-C₂-C₄-alkyl(oxa-C₂-C₄-alkyl)ₙ, mit n = 1 bis 5, -CH₂CH₂-S-CH₂CH₂OH, und Amino-C₂-C₁₂-alkyl, oder Mischungen der Triazine II, und
B) 0,1 bis 10 Mol-%, bezogen auf (A) (a), (A) (b) und (B), unsubstituierten oder mit Resten, ausgewählt aus der Gruppe aus C₁-C₉-Alkyl und Hydroxy, substituierten Phenolen, mit zwei oder drei Phenolgruppen substituierten C₁-C₄-Alkanen, Di(hydroxyphenyl)sulfone oder Mischungen dieser Phenole,
mit
Formaldehyd oder Formaldehyd-liefernden Verbindungen, wobei das Molverhältnis von Melamin, substituierten Melamin I und Triazin II zu Formaldehyd im Bereich von 1:1,15 bis 1:4,5 liegt, zur Herstellung von Fasern und Schaumstoffen.

2. Verwendung nach Anspruch 1, dadurch gekennzeichnet, daß man als substituiertes Melamin I ein mit der 2-Hydroxyethylamino-Gruppe substituiertes Melamin I, ein mit der 2-Hydroxyisopropylamino-Gruppe substituiertes Melamin I, ein mit der 5-Hydroxy-3-oxapentylamino-Gruppe substituiertes Melamin I oder ein mit der 6-Aminohexylamino-Gruppe substituiertes Melamin I einsetzt.

3. Verwendung nach den Ansprüchen 1 bis 2, dadurch gekennzeichnet, daß man ein Phenol, ausgewählt aus der Gruppe aus Phenol, 2,2-Bis(4-hydroxyphenyl)propan und Resorcin, einsetzt.

4. Verwendung nach den Ansprüchen 1 bis 3, dadurch gekennzeichnet, daß man die Komponenten (A) und (B) mit Formaldehyd oder Formaldehyd-liefernden Verbindungen bei 20 bis 150°C und Drücken von 100 bis 500 kPa in Masse oder in einem Lösungsmittel kondensiert.

5. Fasern und Schaumstoffe, erhältlich aus der Verwendung gemäß den Ansprüchen 1 bis 4.

## Claims

1. The use of condensation products obtainable by condensation of a mixture comprising as essential components
(A) from 90 to 99.9 mol% of a mixture consisting essentially of
(a) from 30 to 100 mol% of melamine and
(b) from 0 to 70 mol% of a substituted melamine of the general formula I 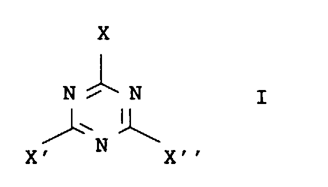 where X, X' and X" are each selected from the group consisting of -NH₂, -NHR and -NRR', and X, X' and X" are not all -NH₂, and R and R' are each selected from the group consisting of hydroxy-C₂-C₁₀-alkyl, hydroxy-C₂-C₄-alkyl(oxa-C₂-C₄-alkyl)ₙ, where n is from 1 to 5, and amino-C₂-C₁₂-alkyl, or mixtures of melamines I, and
c) from 1 to 70 mol%, based on (a) + (b), of a substituted triazine of the general formula II 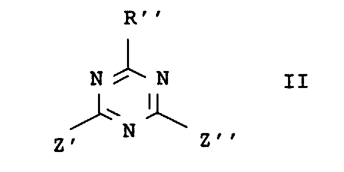 where R" is methyl or phenyl, Z' and Z" are each selected from the group consisting of -NH₂, -NHR^{III} and NR^{III}R^{IV}, and R^{III} and R^{IV} are each selected from the group consisting of hydroxy-C₂-C₁₀-alkyl, hydroxy-C₂-C₄-alkyl(oxa-C₂-C₄-alkyl)ₙ, where n is from 1 to 5, -CH₂CH₂-S-CH₂CH₂OH, and amino-C₂-C₁₂-alkyl, or mixtures of triazines II, and
B) from 0.1 to 10 mol%, based on (A) (a), (A) (b) and (B), of phenols which are unsubstituted or are substituted by radicals selected from the group consisting of C₁-C₉-alkyl and hydroxyl, C₁-C₄-alkanes substituted by two or three phenol groups, di(hydroxyphenyl) sulfones or mixtures of these phenols,
with
formaldehyde or formaldehyde-supplying compounds in a molar ratio of melamine, substituted melamine I and triazine II to formaldehyde within the range from 1:1.15 to 1:4.5, for producing fibers and foams.

2. The use as claimed in claim 1, wherein the substituted melamine I used is a 2-hydroxyethylamino-substituted melamine I, a 2-hydroxyisopropylamino-substituted melamine I, a 5-hydroxy-3-oxapentylamino-substituted melamine I or a 6-aminohexylamino-substituted melamine I.

3. The use as claimed in claims 1 to 2, wherein the phenol used is selected from the group consisting of phenol, 2,2-bis(4-hydroxyphenyl)propane and resorcinol.

4. The use as claimed in claims 1 to 3, wherein the components (A) and (B) are condensed with formaldehyde or formaldehyde-supplying compounds at from 20 to 150°C and pressures from 100 to 500 kPa in the absence of a solvent or in a solvent.

5. Fibers and foams obtainable from the use set forth in claims 1 to 4.

## Revendications

1. Utilisation de produits de condensation, que l'on peut obtenir par condensation d'un mélange contenant, en tant que principaux composants,
(A) de 90 à 99,9% molaires d'un mélange essentiellement constitué de
(a) de 30 à 100% molaires de mélamine et
(b) de 0 à 70% molaires d'une mélamine substituée de la formule générale I 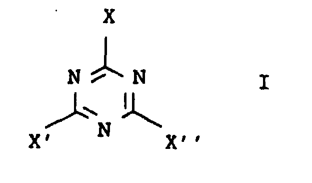 dans laquelle X, X' et X" sont choisis dans le groupe formé par -NH₂, -NHR et NRR', et X, X' et X" ne représentent pas simultanément -NH₂, et R et R' sont choisis dans le groupe formé par les radicaux hydroxyalkyl(C₂-C₁₀), hydroxyalkyl(C₂-C₄)oxaalkyl(C₂-C₄)ₙ, avec n = 1 à 5, et aminoalkyl(C₂-C₁₂), ou des mélanges de mélamines I, et
(c) de 1 à 70% molaires, par rapport à (a) + (b), d'une triazine substituée de la formule générale II 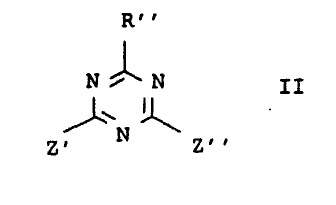 dans laquelle R" représente un radical méthyle ou phényle, et Z' et Z" sont choisis dans le groupe formé par -NH₂, -NHR^{III} et NR^{III}R^{IV}, et R^{III} et R^{IV} sont choisis dans le groupe formé par les radicaux hydroxyalkyl(C₂-C₁₀), hydroxyalkyl(C₂-C₄)oxa-alkyl(C₂-C₄)ₙ, avec n = 1 à 5, -CH₂CH₂-S-CH₂CH₂OH, et aminoalkyl(C₂-C₁₂), ou des mélanges des triazines II, et
B) de 0,1 à 10% molaires, par rapport à (A), (a), (A) (b) et (B), de phénols non substitués ou substitués par des restes choisis dans le groupe formé par des radicaux alkyle en C₁ à C₉ et hydroxy, de phénols substitués, d'alcanes substitués par un ou deux radicaux phénol, de di(hydroxyphényl)sulfones ou des mélanges de ces phénols,
avec
du formaldéhyde ou des composés générant du formaldéhyde, le rapport molaire de la mélamine, de la mélamine substituée I et de la triazine II au formaldéhyde se situant dans une plage de 1:1,15 à 1:4,5, pour la préparation de fibres et de matières expansées.

2. Utilisation selon la revendication 1, caractérisée en ce que l'on met en oeuvre, en tant que mélamine substituée I, une mélamine I substituée par le radical 2-hydroxyéthylamino, une mélamine I substituée par le radical 2-hydroxyisopropylamino, une mélamine I substituée par le radical 5-hydroxy-3-oxapentylamino ou une mélamine I substituée par le radical 6-aminohexylamino.

3. Utilisation selon les revendications 1 et 2, caractérisée en ce que l'on met en oeuvre un phénol choisi dans le groupe formé par le phénol, le 2,2-bis(4-hydroxyphényl)propane et la résorcine.

4. Utilisation selon les revendications 1 à 3, caractérisée en ce que l'on condense les composants (A) et (B) avec du formaldéhyde ou des composés générant du formaldéhyde à des températures de 20 à 150°C et des pressions de 100 à 500 kPa, dans la masse ou dans un solvant.

5. Fibres et matières expansées que l'on peut obtenir par l'utilisation selon les revendications 1 à 4.
